# EUROPEAN PATENT APPLICATION

(11) **EP 0 617 048 A1**
(43) Date of publication of application: **28.09.1994**
(21) Application number: 94104627.8
(22) Date of filing: 23.03.1994
(51) Int. Cl.: C07K 3/16, B01D 57/02, G01N 27/447, G01N 30/80

(54) **Method of capillary isoelectric focusing of proteins and peptides with fraction collection for post-run analysis**

(30) Priority: 26.03.1993 US 37945
(71) Applicant: WATERS INVESTMENTS LIMITED, Wilmington, Delaware 19805 (US)
(72) Inventor: Merion, Michael, Upton, MA 01568 (US); Cheng, Yung-Fong, Taunton, MA 02780 (US)
(74) Representative: Henkel, Feiler, Hänzel & Partner

(57) **Abstract**

A method for performing capillary isoelectric focusing (cIEF) of protein and peptide analytes with fraction collection for the purpose of post separation analysis is disclosed. Use of this method provides a large amount of sample which can be separated and recovered on a porous substrate while preserving the separation efficiency. The large amount of collected sample components may easily be subjected to further analysis such as protein sequencing and amino acid analysis. The cIEF method is conducted by a capillary electrophoresis (CE) system which includes a fused silica capillary, a high voltage power supply, an electrolyte reservoir at one end of the capillary and a porous substrate at the other, means for injecting a sample, and a detector. In carrying out the method, a protein-containing sample is mixed with suitable ampholytes and loaded into the capillary. Subsequent application of the high voltage results in the formation of a pH gradient along the length of the capillary, and more slowly, the migration of the protein analytes to their isoelectric point. The discrete focused zones of analyte are then eluted onto a porous substrate using the bulk fluid flow (electroosmotic flow) or other mobilization techniques associated with the operation of the system. The collected samples become bound to the porous substrate and may then be subjected to further analysis.

## Description

### BACKGROUND OF THE INVENTION

Capillary electrophoresis (CE) and capillary isoelectric focusing (cIEF) are well known separation methodologies which have become techniques of great interest for the separation and analysis of proteins and peptides. These techniques offer the benefits of high resolving power, rapid separations, ability to analyze very small volumes of sample, and a desirable simplicity from the point of view of the apparatus required when compared to competing analytical techniques such as gel electrophoresis and liquid chromatography.

The benefits mentioned above derive to a large extent from the use of narrow diameter capillary tubes, which permit efficient removal of the heat generated in the separation process and prevent convective mixing which would degrade the separating power. The narrow diameter tubes also allow high voltages to be used to generate the electric field in the capillary while limiting current flow and hence heat generation. A CE separation begins by filling the capillary with an electrolyte. Next, a small amount of sample is injected into one end of the capillary. Typical sample injection volumes range from 1-20 nanoliters. After sample injection, a high voltage is applied to the capillary and the sample components are separated based on their charge/mass ratio. In addition, a bulk fluid flow, called electroosmotic flow, moves all components, regardless of charge, through the capillary and generally in a direction that moves them past an appropriate detector such as a UV detector.

Isoelectric focusing is a well known technique for producing high resolution separation of proteins and peptides which originated in gel electrophoresis separations. Over the past years several investigators have shown that isoelectric focusing can also be used as a mode of protein separation using the same hardware as that used for CE separations (Methods Vol 4, pp 1-8, 1992), and the acronym, cIEF, has been adopted to describe this mode of separation. In protein separation, cIEF has been chosen as an analytic technique because of its high resolving power and fast analysis time. As generally practiced, the method mixes the sample with ampholytes, a mixture of small molecules having a heterogeneous range of isoelectric points, at a concentration of between 2 and 5%. The separation capillary is then filled with the mixture prior to the application of the high voltage. This means that 1-2 microliters of sample are loaded into the capillary, in contrast to the nanoliter sample loading typical in CE analyses. Thus, the typical amount of sample that can be loaded using cIEF is 100-1000 times higher than that of CE, which is a direct consequence of how the cIEF method is practiced (i.e., by filling the whole capillary with ampholyte mixture). However, heretofore the primary (and arguably the sole) benefit of the cIEF technique appreciated by those of skill in the protein and peptide analysis field resided in the unique selectivity of the separation.

Continuing with the description of the cIEF method, each end of the capillary is immersed in a buffer electrolyte. The electrolyte at the cathode electrode, termed the catholyte, is generally a basic solution. In some documented methods, 20 mM NaOH is used. The electrolyte in which the anode electrode is immersed, or the anolyte, is generally an acidic solution. In some known methods, 10mM phosphoric acid is used. Upon application of the high voltage, a pH gradient quickly forms along the length of the capillary, and the ampholytes migrate to their isoelectric point. More slowly, the protein analytes also migrate in the pH gradient to their isoelectric points. Finally, in some implementations, the electroosmotic flow moves the gradient with focused protein zones through the tube and past the detector. In other implementations, the focused zones are caused to migrate through the tube by the application of pressure or as a result of a change in the composition of the electrolyte in one of the reservoirs at the end of the capillary.

One of the largest issues in the CE or cIEF separation of analytes is the identification of the separated peaks. In other analytical techniques, such as classical gel electrophoresis or liquid chromatography, fractions from the separation may be readily collected and subjected to further analysis. In liquid chromatography this is generally accomplished by collecting discrete fractions after elution from the column in dry containers, while in gel electrophoresis this can be accomplished by either eluting the separated sample from the gel, or directly transferring the separation from the gel to a porous substrate. This transfer is referred to as blotting, and direct analysis of the blot may also be conducted. However, the collection of discrete liquid fractions from a CE separation is difficult due to the fact that collection must occur in a vial containing electrolyte to maintain electrical continuity. This results in dilution of the sample (often by more than 100 fold), and the frequent loss of the dilute material which can adhere to the walls of the fraction collection container. In addition, because CE permits the injection of only a few nanoliters of sample, the problem of fraction collection is exacerbated when dealing with low sample concentrations e.g., only up to about 1 mg/ml. In this case, the typical amounts of sample separated are generally less than 1 ng (which amounts to less than 1 pmole of a 1000 dalton peptide). This provides amounts of CE separated analytes that are often inadequate for post-run analysis such as protein sequencing.

Nonetheless, uses of fraction collection for the purpose of further analysis of sample components following a CE separation have been described. Camilleri et. al. (Anal. Biochem. Vol 196, pp 178-182, 1991) used D₂O-based instead of H₂O-based electrolyte solution to minimize the diffusion of the analyte and then eluted the separated components through pressure to facilitate fraction collection. Huang and Zare (Anal. Chem. Vol 62, pp 443-446, 1992) used an on-column sintered glass frit structure which made it possible to ground a capillary prior to its outlet. Electroosmotic flow in the capillary causes transport of the separated species to the capillary outlet and thus permits sample collection at the exit end of the capillary. Fujimoto et al. (J. of High Resol. Chromatogra., Vol 15, pp 201-203, 1992) used an on-column fracture, which is surrounded by polyacrylamide gel, to facilitate the CE fraction collection. Again, the electric circuit was completed prior to the capillary outlet. Each of these techniques result in the recovery of only a relatively small amount of sample and are not particularly suited for use in protein sequencing and amino acid analysis. Moreover, disrupting the capillary electrical circuit prior to the outlet of the tube has the effect of truncating the charge induced motive force which results from the application of high voltage. Instead, movement of sample rests with bulk flow properties and in some applications this bulk flow rate is low enough to present difficulties in achieving full separation.

Another approach for CE fraction collection is described in U.S. Patent No. 5,126,025 wherein a porous substrate assembly is used to effectively complete the electric circuit at the outlet end of the capillary through a collection membrane disposed on the substrate. The separated components are continuously collected by a rotating disk or a moving belt. This technique, which directly deposits the analytes onto a moving porous membrane, represents an advance over prior attempts at fraction collection as it ensures 100% deposition, minimizes dilution, maintains the full spatial resolution of the separation, and continues the electrical circuit through the end of the entire capillary. The porous substrate to which the analytes have been bound can be subjected to analysis using a variety of antibody labeling techniques. Further, protein sequencing may also be conducted directly from the sample bound to the porous membrane. However, experience has found that protein sequencing is sometimes difficult with the amount of sample that can be collected from a single CE separation using the apparatus described in this patent. For example, typical minimum requirements for a 10,000 dalton protein are about 10 pmoles of sample for sequencing, and a typical CE separation employing the techniques disclosed in U.S. Patent No. 5,126,025 can deposit only about 0.1 pmoles on a porous membrane.

Thus the need still exists for a technique of separating and collecting protein-containing analytes involving the use of capillary electrophoresis in quantities sufficient for post-separation analysis, such as protein sequencing or amino acid analysis. It would be desirable to collect such analytes on a porous substrate with minimum dilution and minimum disruption of the motive forces which propel the sample through the capillary.

### SUMMARY OF THE INVENTION

The present invention provides a protein or peptide separation and collection process and apparatus which employs capillary isoeletric focusing (cIEF) and fraction collection for the purpose of post-separation analyses such as amino acid analysis and protein sequencing. This invention provides a simple, efficient, high-speed, and high-throughput methodology for effectively collecting large amounts of separated sample analytes for further analysis while still preserving the cIEF spatial resolution.

In accordance with a preferred embodiment of this invention, a CE system is used to perform a cIEF separation, and the separated analytes are deposited, for fraction collection, on a porous membrane substrate. The capillary used is made of fused silica and has an I.D. of from 1-500 µ m, an O.D. of from 100-1000µ m and length of from 5-200 cm. After the capillary is filled with a mixture of protein-containing sample and ampholyte, the anolyte end is immersed in an electrolyte of 10mM phosphoric acid. The catholyte end is in direct contact with a porous membrane which is wet with a second electrolyte, 20 mM sodium hydroxide. High voltage is applied through the capillary to effect movement of the sample toward the porous membrane. The sample components are adsorbed onto the porous membrane, while the ampholytes and other buffer components used in the isoelectric focusing can be easily washed away from the substrate to ensure that there is no interference with any post-run analysis.

Other aspects, advantages and objectives of the present invention will become apparent from the following detailed description taken in conjunction with the accompanying drawings.

### BRIEF DESCRIPTION OF THE DRAWINGS

Figure 1 shows a block diagram of a CE system and fraction collection device of the present invention.

Figure 2 shows an electropherogram of the separation of cytochrome c using isoelectric focusing collected onto a porous membrane substrate.

Figure 3 is a photograph of a typical separation of cytochrome c using the method of this invention showing the location of the cytochrome c band on the porous membrane substrate after CE fraction collection.

### DETAILED DESCRIPTION OF THE INVENTION

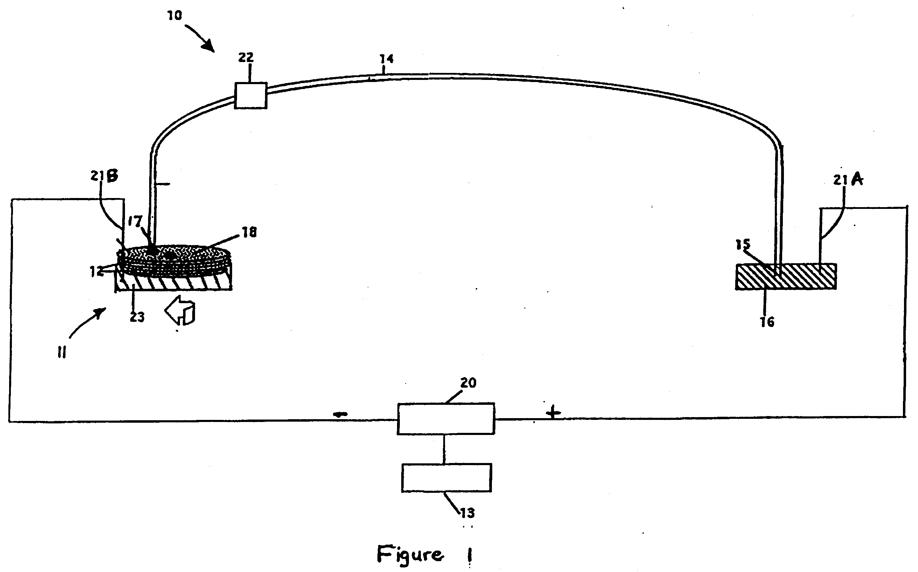

Figure 1 shows a block diagram of a CE system 10 that is linked to a fraction collector 11 that employs a porous substrate 12 to keep a porous membrane 18 positioned on the substrate wet with electrolyte which is contained in an outlet reservoir 23. The system includes a fused silica capillary 14 with an inside diameter having a range of about 5 to 500µm, an outside diameter having a range of about 100 to 1000µm and a length having a range of about 5 to 200 cm. The capillary includes an inlet end 15 immersed in an inlet reservoir 16 containing electrolyte and an outlet end 17 in direct contact with the porous membrane 18. The inside of the capillary is filled in known fashion with a protein or peptide sample immersed in a mixture containing carrier ampholytes, the details of which will be described subsequently. Contacting the reservoirs 16, 23 are separate electrodes 21A, 21B that are connected to the output terminals of a high voltage power supply 20. The electrical circuit is completed from the input electrode 21A through the filled capillary to the outlet end of the capillary 17 through the porous membrane 18, through the substrate 12 and to the high voltage electrode 21B that is immersed in the outlet reservoir 23. The high voltage power supply output is set by a controller 13. The separation is monitored using an on-line UV detector 22.

The fraction collector 11 includes the outlet reservoir 23, filled with a suitable electrolyte, the porous substrate 12 and the porous membrane 18.

The electrode 21B is immersed in the outlet reservoir and the electrolyte contained therein provides continuous wetting of the substrate and the membrane to maintain electrical conductivity while sample elutes from the outlet end 17 and is collected on the membrane. In this embodiment, the fraction collector is configured to effect rotational movement as shown by the arrow in Figure 1 so that a pattern of the separated sample is formed on the surface of the membrane. However, it is also possible to move the collector translationally to achieve the same effect. Moreover, it may be desireable in certain instances to keep the fraction collector stationary so that discrete samples may be individually collected.

The apparatus thus described is similar in many respects to that set forth in U.S. Patent No. 5,126,025, whose description is thus incorporated by reference as if fully set forth herein.

In one embodiment, to perform a cIEF separation and fraction collection on the porous membrane 18, the capillary 14 is filled with a protein sample mixed with an ampholyte solution at a concentration of between 2 and 5%. The inlet end 15 of the capillary is immersed in the reservoir 16, containing as the anolyte buffer an acidic solution such as 10 mM phosphoric acid, and the outlet end 17 of the capillary is in direct contact with porous membrane 18. This membrane is kept wet with the catholyte buffer placed in the reservoir 23, which is generally a basic solution such as 20 mM NaOH. The membrane 18 is in direct contact with the substrate 12 also located in the buffer reservoir 23, and the capillary 14 is grounded through this buffer reservoir 23. The high voltage electrodes 21A, 21B, placed in the reservoirs 16, 23 respectively, are connected to the high voltage power supply 20.

When the high voltage power supply 20 is turned on, a pH gradient is quickly formed by the ampholytes. More slowly, the proteins are focused within the pH gradient. While the proteins are focusing, a slow bulk fluid flow is created in the capillary tube. In one embodiment, this force is a result of the electroosmotic flow, which moves the entire contents of the capillary 14 towards the porous membrane 18. Alternatively, the force may be the result of the application of pressure, or the result of changing the formulation of the catholyte and anolyte buffers. While the separation is taking place, the porous membrane 18 slowly moves while maintaining direct content with the outlet end 17 of the capillary. This results in a continuous deposition of the capillary effluent on the membrane, maintaining the spatial resolution of the cIEF separation. Continued application of the high voltage eventually results in the deposition of all of the focused proteins on the surface of the moving porous membrane 18. As the analytes exit the capillary, they are bound tightly to the porous membrane.

The porous membrane 18 may comprise a microporous or ultrafiltration membrane, both being either hydrophilic or hydrophobic, or can comprise a gel such as an aqueous gel or a polyacrylamide membrane. It may be advantageous to use a membrane which is hydrophobic when it is dry and which is hydrophilic when it is wet to complete the electric circuit. Representative membranes capable of binding proteins from solution are well known to those of skill in the art. However, presently preferred membranes include those made of polyvinylidene difluoride or nylon.

The following example illustrates the present invention and is not intended to limit the same.

### EXAMPLE

For this experiment, cIEF/fraction collection of protein-containing analytes was done using a continuously rotating collecting porous membrane substrate disk with a polyvinylidene difluoride (PVDF) membrane (Immobilon™-P commercially available from Millipore Corporation, Bedford, MA) placed on the disk as the collection media. The capillary had a 75 µm i.d., 365 µm o.d. and a length of 60 cm. An on-line UV detector was employed at a wavelength of 280 nm. The detection region was 8.8 cm from the exit end of the capillary. The fraction collection assembly was rotated at 31.24 min per revolution. A voltage of 18 KV was applied across the capillary. The anolyte was 10 mM phosphoric acid, and the catholyte was 20 mM sodium hydroxide.

The protein sample used was a 2.9 mg/ml solution of cytochrome c (sold by Sigma Chemical Company as Part No. C7752). This solution was mixed with ampholytes commercially available from Millipore Corporation and optimized for 2D-electrophoresis, pH 3-10/2D, (identified as Catalogue No. ELCR1DC10) at 5%, 0.1 % Methyl Cellulose (4,000 cp viscosity at 2%), and 1% of TEMED.

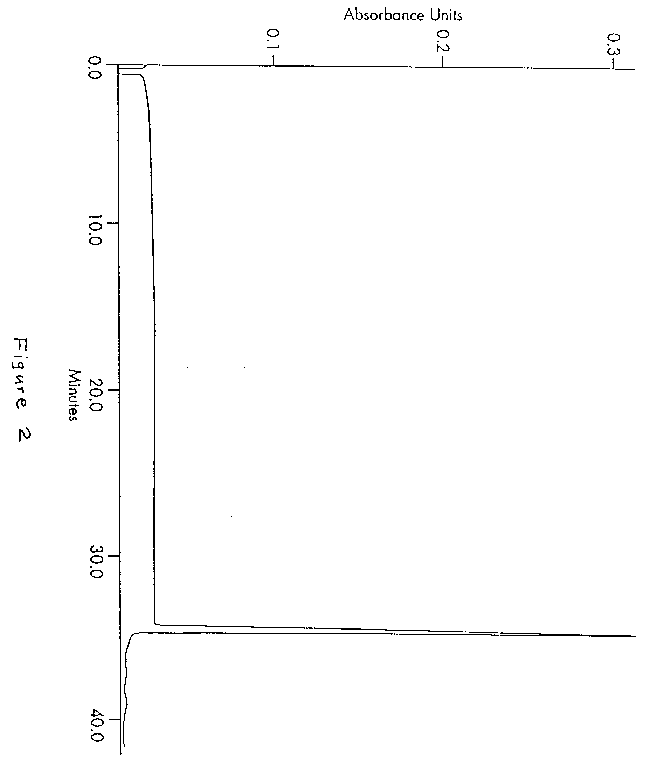

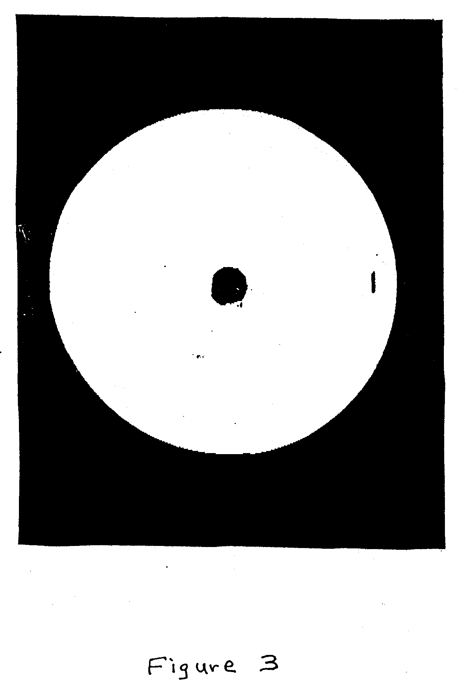

The UV detector output of the separation of cytochrome c shown in Figure 2 reveals that no peaks eluted from the capillary for approximately the first 30 minutes of analysis run time but that one peak was observed and the migration time was at 34.4 min. This peak corresponds to the quantity of cytochrome c sample that was separated from the mixture by the CE system. In practice, this sample is collected on the membrane in a discrete zone or band. Figure 3 shows a typical separation of cytochrome c as collected on the membrane, which is catalogued by identifying number. The location on the membrane after CE fraction collection appears as a vertical band to the right of the center hole near the periphery of the membrane. As shown, only one elongated spot is clearly visible.

This spot or band was then excised from the PVDF membrane and subjected to acid hydrolysis, followed by an AQC-Derivatization procedure, the details of which may be determined from pending U.S. Patent Application Serial No. 07/762,579, filed September 9, 1991, the disclosure of which is incorporated herein by reference. The labeled amino acids were separated and identified using high performance liquid chromatography (HPLC). The amino acid analysis is shown in Table 1. In addition, cytochrome c from the manufacturer's vial was spotted onto a PVDF membrane with a pipette. The membrane was subjected to acid hydrolysis, the same AQC derivitization procedure described above, and the labeled amino acids were identified using HPLC. The amino acid analysis is shown in Table 2. This analysis was used as a positive control. Finally, a blank membrane was processed in the same manner and used as a negative control. The resultant amino acid analysis is shown in Table 3.

The amino acid analysis shown in Table 1 derived from the cIEF separation according to the method of this invention is very similar both to the known composition of cytochrome c, as well as to the positive control seen in Table 2. An average error of 12.2% was obtained for the analysis in Table 1, which is only slightly worse than the average error of 9.0% obtained for the positive control in Table 2. Because average errors of up to 20% are common for proteins that are analyzed from membranes, this analysis demonstrates that sufficient sample can be deposited onto a membrane in a single cIEF separation to yield meaningful amino acid data. The blank control membrane amino acid analysis seen in Table 3 demonstrates the low background of such a technique, further verifying the utility of the methodology.

Although the link of the cIEF and membrane fraction collection for amino acid analysis has been demonstrated, it is apparent that the method will have applicability to other post separation analytical techniques and fraction collection apparatus. Other variations of this invention will suggest themselves to people with ordinary skill in the art.

For example, the invention has been described for use with amino acid analysis of the collected proteins and peptides; however, the principles demonstrated are equally useful for protein sequencing, laser assisted desorption for mass spectrometer analysis, or antibody linked staining of proteins or peptides.

In addition, the capillary isoelectric focusing techniques described herein may be performed using a variety of capillaries, ampholytes, and buffers. The example cited above uses uncoated fused silica capillaries, and relies on the electroosmotic flow for movement or mobilization of the sample through the capillary. Salt mobilization techniques in conjunction with the use of a coated capillary may also be employed. The addition of salt to either buffer causes mobilization of the gradient in a coated capillary with a suppressed electroosmotic flow. It is also possible to use similar coated capillaries with vacuum mobilization. Mobilization may be conducted as part of a two step process using a coated capillary tube. In the first step, isoelectric focusing occurs; thereafter, the buffer positions are reversed, resulting in mobilization of the gradient.

**Table 1**

| Amino Acid | Cytochrome c (Known Composition) | Cytochrome c (Composition Derived From Membrane) |
|---|---|---|
| Asp | 8 | 8.34 |
| Glu | 12 | 12.52 |
| Gly | 12 | 12.91 |
| His | 3 | 0.52 |
| Arg | 2 | 2.03 |
| Thr | 10 | 9.83 |
| Ala | 6 | 6.97 |
| Pro | 4 | 4.22 |
| Tyr | 4 | 2.83 |
| Val | 3 | 3.28 |
| Met | 2 | 0.95 |
| Lys | 19 | 14.17 |
| Ile | 6 | 5.91 |
| Leu | 6 | 6.14 |
| Phe | 4 | 3.95 |
| Average error = 12.2% | | |

**Table 2**

| Amino Acid | Cytochrome c (Known Composition) | Cytochrome c (Composition Derived From Membrane) |
|---|---|---|
| Asp | 8 | 9.21 |
| Glu | 12 | 12.97 |
| Gly | 12 | 12.06 |
| His | 3 | 2.76 |
| Arg | 2 | 2.00 |
| Thr | 10 | 9.14 |
| Ala | 6 | 6.25 |
| Pro | 4 | 3.76 |
| Tyr | 4 | 4.47 |
| Val | 3 | 3.36 |
| Met | 2 | 1.02 |
| Lys | 19 | 17.59 |
| Ile | 6 | 5.92 |
| Leu | 6 | 6.10 |
| Phe | 4 | 3.83 |
| Average error = 9.0% | | |

**Table 3**

| Amino Acid | Cytochrome c (Known Composition) | Cytochrome c (Composition Derived From Membrane) |
|---|---|---|
| Asp | 8 | -- |
| Glu | 12 | -- |
| Gly | 12 | 0.62 |
| His | 3 | -- |
| Arg | 2 | -- |
| Thr | 10 | -- |
| Ala | 6 | -- |
| Pro | 4 | -- |
| Tyr | 4 | -- |
| Val | 3 | -- |
| Met | 2 | -- |
| Lys | 19 | -- |
| Ile | 6 | -- |
| Leu | 6 | -- |
| Phe | 4 | -- |

## Claims

1. A method for separating and collecting sample protein or peptide species comprising the steps of:
a. filling the inside of a capillary with a mixture of sample and ampholytes;
b. immersing a first inlet end of the capillary in a first electrolyte;
c. placing a second outlet end of the capillary in contact with a porous substrate;
d. continuously wetting the porous substrate with a second electrolyte;
e. placing the porous substrate in contact with the second electrolyte;
f. contacting the first and second electrolytes with electrodes connected to output terminals of a high voltage power supply;
g. applying a high voltage potential across the ends of the capillary to allow the ampholytes to form a pH gradient within the capillary and to, allow the sample to be focused within the pH gradient;
h. producing a flow within the capillary to move the contents of the capillary towards the porous substrate; and
i. collecting the sample portion of the contents of the capillary onto the porous substrate.

2. The method of claim 1 wherein the porous substrate is stationary.

3. The method of claim 1 wherein the porous substrate is in motion with respect to the outlet end of the capillary.

4. The method of claim 3 wherein the porous substrate rotates with respect to the outlet end of the capillary.

5. The method of claim 1 wherein said porous substrate includes a microporous membrane.

6. The method of claim 1 wherein said porous substrate includes an ultrafiltration membrane.

7. The method of claim 5 wherein said membrane is a nylon membrane.

8. The method of claim 5 wherein said membrane is a polyvinylidene fluoride membrane.

9. The method of claim 6 wherein said membrane is a nylon membrane.

10. The method of claim 6 wherein said membrane is a polyvinylidene fluoride membrane.

11. The method of claim 1 wherein the collected sample is used for the purpose of amino acid analysis of collected proteins and peptides.

12. The method of claim 1 wherein the collected sample is used for the purpose of protein sequencing of collected proteins and peptides.

13. The method of claim 1 wherein the collected sample is used for the purpose of laser assisted desorption for mass spectrometer analysis.

14. The method of claim 1 wherein the collected sample is used for the purpose of antibody linked staining of proteins or peptides.

15. The method of claim 1 wherein said first electrolyte is an anolyte comprising phosphoric acid and said second electrolyte is a catholyte comprising sodium hydroxide.

16. The method of claim 1 wherein said mixture of sample and ampholytes is caused to flow within said capillary by electroosmotic flow.
